(19) Europäisches Patentamt
European Patent Office
Office européen des brevets

(11) Publication number: 0 419 077 A2

(12) EUROPEAN PATENT APPLICATION

(21) Application number: 90309581.8

(51) Int. Cl.5: C07C 43/13, C07C 41/06

(22) Date of filing: 31.08.90

(30) Priority: 20.09.89 US 410168

(43) Date of publication of application: 27.03.91 Bulletin 91/13

(84) Designated Contracting States: DE ES FR GB

(71) Applicant: TEXACO CHEMICAL COMPANY
3040 Post Oak Boulevard
Houston, Texas 77056(US)

(72) Inventor: Knifton, John Frederick
10900 Catskill Trail
Austin, Texas 78750(US)

(74) Representative: Brock, Peter William et al
URQUHART-DYKES & LORD 91 Wimpole Street
London W1M 8AH(GB)

# (54) Synthesis of low molecular weight ethylene propylene glycol ethers via olefin addition to the corresponding glycol.

(57) A selective process for the synthesis of ethers of polyols involves reacting an olefin and a polyol in the presence of a heteropoly acid catalyst at a temperature of 25 to 200°C and a pressure of up to 7MPa.

EP 0 419 077 A2

## SYNTHESIS OF LOW MOLECULAR WEIGHT ETHERS

This invention relates to the synthesis of glycol monoalkyl ethers, and more particularly this invention relates to a process for the synthesis of ethylene and propylene glycol monoalkyl ethers from low molecular weight olefins and the corresponding glycol using a heteropoly acid catalyst. This is a new route to these products and the invention is particularly advantageous in that the desired monoalkyl ethers have been prepared in high selectivity with good glycol conversion levels. In addition, the process can take place over a range of conditions. There is an expanding market for propylene and butylene glycol monoalkyl ethers because they can be substituted for ethylene glycol monomethyl ether and ethylene glycol monoethyl ether in solvent formulations, since the latter have been determined to be carcinogenic.

Low molecular weight glycol ethers and their acetate derivatives are widely used as solvents in paints and coatings. In 1984, the EPA issued guidelines to reduce exposure to 2-methoxyethanol, 2-ethoxyethanol and their acetates since studies indicated these chemicals were carcinogens.

Ethylene glycol monobutyl ether plays an important role in waterborne coatings, accounting for 80% of glycol ether content, but has not been implicated in any studies as a tetrogen. Many solvents have been reformulated in recent years to use heavier ethylene glycol and propylene glycol monobutyl ethers rather than lighter ethylene glycol monomethyl and ethyl ethers (see for example European Patent Application 288-856A).

Methods of preparing ethylene and propylene glycol mono-n-butyl ethers are known in the art. For example, one method for preparation is by the reaction of ethylene oxide or propylene oxide and n-butanol.

Another method of synthesizing low molecular weight ethers is disclosed in U. S. Patent No. 4,714,787 which discloses a process for selectively reacting one or more linear monoolefins with a primary or secondary lower molecular weight alcohol to form the corresponding ethers. The active acidic catalyst component is a sulfonate ion-exchange resin or a crystalline silicate having a pore size greater than 5 A.U. In the preferred embodiment, methanol and propylene are reacted to selectively form methyl isopropyl ether and the preferred crystalline silicate includes a crystalline zeolite having a silica to alumina mole ratio greater than about 12. Here the main products are methyl isopropyl ether and dimethyl ether.

In U. S. Patent No. 4,675,082 there is disclosed a method for preparation of 1-t-butoxy-2-propanol from the etherification of propylene glycol with isobutylene in the presence of a solid resin etherification catalyst, typically an acidic ion exchange resin such as AMBERLYST®-15, consisting of sulfonated polystyrene matrix having up to about 25% of copolymerized divinylbenzene tearing functional nuclear sulfonic acid groups contained therein. This process requires a two tower separation.

Polyethylene glycol and propylene have been reacted to form polyethylene glycol dipropyl ethers using a strongly acidic cation resin as a catalyst. This process provides good yields of polyethylene glycol dipropyl ethers by reducing side reactions such as polymerization and decomposition. See reference (J58052321-A or J8 8037819-B) in Derwent Japanese Patents Abstract, Polymer + General Chemistry, Vol. 88, No. 30. In a Japanese reference to the same company, Nippon Shokubai Kagaku, polyethylene glycol is reacted with isobutylene to produce polyethylene glycol dibutyl ether (J58049725-A).

The preparation of propylene glycol tert-butyl ether from propylene glycol and isobutylene is disclosed in Jpn. Kokai Tokkyo Koho JP 63,250,336 to Fujiwara, Hiroshi et al. (Maruzen). A strongly acidic cation-exchanger resin is employed along with tert-butanol ($Me_3COH$) which serves to inhibit the formation of byproduct diisobutylene. The reaction product must be distilled to separate propylene glycol tert-butyl ether from propylene glycol di-tert butyl ether.

In an article titled "Advances in Catalysis By Heteropolyacids", by Kozhevnikov, in Russian Chemical Reviews , v. 56, No. 9, pp. 811-825 (September 1987) there is a discussion of the acid properties of heteropolyacids in solutions and in the solid state, their proton structure, and the characteristic features of the homogeneous and heterogeneous acid catalyses. On page 817 of this article there is a list of acid catalyses in which heteropolyacid catalysts have been employed.

In an article titled "Heterogeneous Catalysis By Heteropoly Compounds Of Molybdenum And Tungsten", Misona discusses characteristics of heteropoly compounds which made them advantageous as heterogeneous catalysts, including design, molecularity and reaction field properties. There is also a discussion of why heteropoly acids are much stronger than oxoacids of constituent elements and ordinary mineral acids and how they are more active than ordinary solid acids such as zeolites and silica-alumina. It is reported that heteropoly compounds are efficient for reactions of oxygen-containing molecules such as dehydration, etheration, esterification and related reactions at relatively low temperatures. Catal. Rev.-Sci. Eng., 29 (2 & 3), 269-321 (1987).

There does not appear to be any art suggesting the selective synthesis of low molecular weight

monoalkyl ether derivatives of ethylene and propylene glycol via olefin addition to the corresponding glycol by use of a heteropoly acid catalyst such as those containing tungsten or molybdenum.

It would be a distinct advance in the art if a process were available which allowed for the continuous production of low molecular weight glycol monoalkyl ethers in high selectivity and conversion from readily available, and inexpensive, glycol/olefin feedstock combinations. Furthermore, it would be advantageous if this reaction was one-step, it did not require any second distillation and the catalyst was physically and chemically stable to the reactant/product media over a broad range of temperatures, particularly above 100 °C, so that there are minimal problems with catalyst decomposition and loss of activity.

It has been surprisingly discovered that heteropoly acid catalysts can be used to synthesize ethylene and propylene glycol ethers from the corresponding olefins and glycols. It is an object of the present invention to recover ethylene and propylene glycol ethers in a high state of purity with minimal amounts of by-products. Other objects will become apparent to those skilled in the art from the following description.

In accordance with the foregoing, the novel method of this invention for synthesis of polyol ethers comprises reacting low molecular weight olefins and the corresponding polyol in the presence of a catalyst comprising a heteropoly acid.

The desired glycol monoalkyl ethers have been prepared with selectivity as high as >99%, at glycol conversion levels of ca. 50%. Both batch and continuous synthesis can be used.

Preparation of the glycol products of this invention may be carried out preferably by reacting ethylene or propylene glycol with propylene or isobutylene batchwise, or continuously, in the presence of a catalyst comprising a heteropoly acid on a support. The products include propylene glycol mono-t-butyl ether, propylene glycol mono-isopropyl ether, ethylene glycol mono-t-butyl ether and ethylene glycol mono-isopropyl ether.

The general synthesis is illustrated by the following equation:

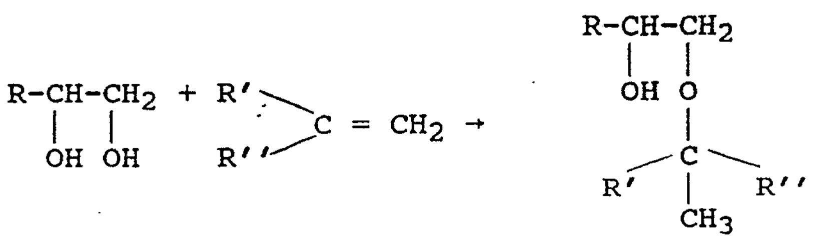

(Eq. 1)

where R, R′, R″ may be hydrogen or an alkyl radical.

Both linear and isoalkenes, as well as mixtures thereof, are useful in the process of this invention, including those having 2 to 6 carbon atoms and the structure:

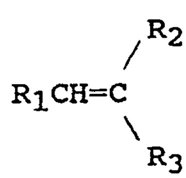

wherein $R_1$, $R_2$ and $R_3$ individually are hydrogen or alkyl groups and the total carbon atoms in $R_1 + R_2 + R_3$ is from 0 to 4. The preferred linear or isoalkenes are those having 3 to 5 carbon atoms, i.e. the total carbon atoms in $R_1 + R_2 + R_3$ is 1 to 3. These olefins include propylene, 1-butene, isobutylene, 1-pentene, 2-pentene, 2-methyl-1-butene and isoamylene. Particularly preferred are prcpylenc and isobutylene. Said olefins may be employed in diluted form, e.g. the isobutylene may be contained in a typical $C_4$ stream (from an ethylene plant).

The glycols which are useful in the practice of this invention are generally diols having 2 to 20 carbon atoms per molecule. They may include vicinal glycols where the hydroxyl groups are bonded to adjacent carbon atoms, as in Eq. 1, or alternatively, the hydroxyl groupings may be separated by additional carbon atoms, as in the case of 1,3-propylene glycol and 1,6-hexane diol. Alternatively, said substrates may contain three or more hydroxyl groups per molecule, as in the case of glycerine, or they may be a polyalkylene glycol, particularly a polyethylene glycol or a polypropylene glycol containing multiple ether linkages and terminal hydroxyl groups. Said polyalkylene glycols may have molecular weights in the range of from ca. 150 to ca. 6000, and be mixtures of different polyethylene glycol or polypropylene glycol oligomers.

Furthermore they may be capped or contain within the backbone, certain higher molecular weight carbon units, such as the $C_4$-unit, introduced during oligomerization by the addition of, for example, 1,2-butylene oxide or isobutylene oxide.

The preferred glycols for the generation of glycol monoalkyl ethers using heteropoly acid catalysts are vicinal glycols containing 2 to 20 carbon atoms per molecule. These include ethylene glycol, 1,2-propylene glycol, 1,2-butylene glycol, diethylene glycol and triethylene glycol.

The molar ratio of said glycols and olefins in the feed mixture may vary widely, from at least 100:1 to 1:100. To achieve optimum selectivities and yields of desired glycol monoalkyl ethers (Eq. 1), it is desirable that the feed should be rich in glycol component, i.e. the molar feed ratio of glycol-to-olefin should be greater than unity. On the other hand, if it is desirable to make glycol dialkyl ethers, then the feed should be rich in olefin and the molar ratio should be less than unity. These conditions are illustrated in the accompanying examples.

The catalysts used to effect this reaction are preferably heteropoly acids. The catalysts are effective both as homogeneous catalysts, solubilized in the reactants, added solvent, or combinations thereof, and as heterogeneous catalysts, where the heteropoly acids are bonded to an inert support.

The heteropoly acids that are catalysts in the subject reaction comprise a class of acids formed by the condensation of two or more inorganic oxyacids. For example, phosphate and tungstate ions, when reacted in an acidic medium, are condensed to form 12-tungstophosphoric acid, a typical heteropoly acid (HPA) according to Equation 2:

$$PO_4^{3-} + 12WO_4^{2-} + 27H^+ \rightarrow H_3PW_{12}O_{40} + 12H_2O \quad \text{(Eq. 2)}$$

A wide variety of elements ranging from Group I to Group VIII can become the central atom of the HPA anion, or the heteroatom as it is called (P in the case of Eq. 2). The nature of the heteroatom is a governing factor which determines both the condensation structure and the physical properties of the HPA.

Atoms coordinated to the heteroatom via oxygens are called polyatoms (W in the case of Equation 2) and in most cases are any one of such limited species as molybdenum, tungsten, niobium and vanadium. In the case of molybdenum (Mo) as the polyatom, the nature of the heteroatoms, condensation ratios and chemical formulae of the corresponding HPA anions are summarized in Table I.

Anions containing the so-called Keggin structure have a condensation ratio of 1:12 and are the most typical of all HPA anions. Heteropoly acids with the Keggin structure, and their homologues, are generally the most readily available HPA's and the ones most commonly used in catalysis. The synthesis of these HPA's is well documented in the literature [see for example U. S. patent 3,947,332 (1976)].

TABLE 1

| Typical heteropolymolybdate anions | | | |
|---|---|---|---|
| CONDENSATION RATIOS | | HETERO ATOMS(X) | CHEMICAL FORMULAS |
| 1:12 | Keggin structure | $P^{5+}$, $As^{5+}$, $Si^{4+}$, $Ge^{4+}$ | $[X^{n+}Mo_{12}O_{40}]^{-(8-n)}$ |
| | Silverton structure | $Ce^{4+}$, $Th^{4+}$ | $[X^{4+}Mo_{12}O_{42}]^{8-}$ |
| 1:11 | Keggin structure (decomposition) | $P^{5+}$, $As^{5+}$, $Ge^{4+}$, $Si^{4+}$ | $[X^{n+}Mo_{11}O_{39}]^{-(12-n)}$ |
| 2:18 | Dawson structure | $P^{5+}$, $As^{5+}$ | $[X_2^{5+}Mo_{18}O_{62}]^{6-}$ |
| 1:9 | Waugh structure | $Mn^{4+}$, $Ni^{4+}$ | $[X^{4+}Mo_9O_{32}]^{6-}$ |
| 1:6 | Anderson structure (A type) | $Te^{6+}$, $I^{7+}$ | $[X^{n+}Mo_6O_{24}]^{-(12-n)}$ |
| | (B type) | $Co^{3+}$, $Al^{3+}$, $Cr^{3+}$ | $[X^{n+}Mo_6O_{24}H_6]^{-(6-n)}$ |
| 4:12 | | $As^{5+}$ | $[H_4As_4Mo_{12}O_{52}]^{4-}$ |
| 2:5 | | $P^{5+}$ | $[P_2Mo_5O_{23}]^{6-}$ |

In the case of olefin addition to the corresponding glycol to form glycol ethers suitable heterpoly acid catalysts may contain polyatoms selected from the group molybdenum, tungsten, niobium and vanadium, while the heteroatoms may be phosphorus, silicon, germanium, and arsenic. Preferably the heteroatoms are phosphorus or silicon. These heteropoly acids would likely have the Keggin structure, $H_{8-n}[XM_{12}O_{40}]$, where X = P or Si, M = Mo or W and n is an integer which is 4 or 5.

The preferred heteropoly acids for the practice of this invention include 12-molybdophosphoric acid, $H_3PMo_{12}O_{40}$, 12-tungstophosphoric acid, molybdosilicic acid, $H_4SiMo_{12}O_{40}$ and 12-tungstosilicic acid. Said acids are generally used as their hydrates; they may be employed by themselves, partially or completely dissolved in the glycol feed, or they may be employed as heterogeneous catalysts bonded to a suitable

support.

The support should preferably comprise an inert compound. Compounds which may be employed are those containing elements of Group III and IV of the Periodic Table. Suitable compounds include the oxides of aluminum, silicon, titanium and zirconium or combinations thereof, e.g. alumina, silica (silicon dioxide), titania (titanium dioxide) and zirconia, as well as combinations thereof. Also suitable are carbon, ion-exchange resins and carbon-containing supports. Good results were observed using $TiO_2$ as the support.

The inert support may be in the form of powders, pellets, spheres, shapes and extrudates. As will be demonstrated by the examples, the supports are preferably of high purity and high surface area. It has been found in the process of this invention that greater conversion of glycol and olefin feed is achieved where the surface area of the support is generally >10 $m^2/g$.

The weight percent of heteropoly acid to Group III/Group IV support should be such that the concentration of the polyatom (Mo, W, Nb or V) in the formulated catalyst is in the range of 0.1 wt% to 30 wt%, although concentrations outside this range may also be employed. Where the heteropoly acid is, for example, 12-molybdophosphoric acid, supported on titania, a suitable quantity of molybdenum is 1-10 wt%. In the preparation of a tungstophosphoric acid-on-titania catalyst, on the other hand, the tungsten content may be 1-30 wt%.

A solvent may optionally be added to facilitate the desired glycol ether synthesis. Suitable solvents include polar organic solvents containing one or more ether linkages or non-polar polyether solvents. Examples of satisfactory solvents include p-dioxane, 1,3-dioxane, 1,3-dioxolane, triglyme and tetraglyme. Glycol monoalkyl ethers may also serve as suitable solvents for reaction 1; this includes the product glycol monoalkyl ethers such as ethylene glycol t-butyl ether and the propylene glycol mono-t-butyl ethers.

The low molecular weight ether synthesis may be conducted batchwise, in a continuous slurry bed reactor, or in a fixed-bed, continuous flow, reactor. For practical reasons a fixed bed process is preferred.

Synthesis of ethylene and propylene glycol ethers can generally be conducted at temperatures from 0° to 300° C; the preferred range is 25° to 200° C. The operating pressure may be from zero to 1000 psig, or higher. The preferred pressure range is 100 to 400 psig. Optimum conditions may vary. The synthesis of ethylene glycol t-butyl ether from ethylene glycol plus isobutylene preferably takes place at 80-120° C, whereas ethylene glycol isopropyl ether is prepared at higher temperatures, around 180° C. In the latter case, the concentrations of monoether in the crude product are lower. At 100° C, propylene glycol monobutyl ethers are produced in total selectivities of about 92 to >99 mole%.

The principal ether products produced depend upon the olefin and glycol charged. In the case of ethylene glycol, the addition of propylene results in ethylene glycol mono-isopropyl ether as the principal product, while the addition of isobutylene results in ethylene glycol mono-t-butyl ether plus a smaller amount of 1,2-di-t-butoxyethane. When the substrate is 1,2-propylene glycol, the addition of isobutylene results primarily in the formation of 1-t-butoxy-2-propanol plus lesser amounts of 2-t-butoxy-1-propanol and 1,2-dibutoxypropane. The majority monoalkyl glycol ether products are formed in accordance with the Markovnikov rule of addition to the double bond of a linear olefin and involve the primary (rather than the secondary) hydroxyl group of the glycol substrate.

The resultant reaction mixture, containing the desired glycol ether product, undesired by-products and unreacted reactants such as glycol, are normally separated in a conventional manner by distillation.

Typically, monoalkyl ethers are generated continuously in up to ca. 92% to >99% selectivity and at glycol conversion levels of ca. 50%.

These yields are achieved at total liquid hourly space velocities (LHSV) of 1 to 5 under mild conditions. LHSVs of 5, or greater, have also been demonstrated to be useful in achieving satisfactory olefin conversion levels.

Here LHSVs is defined as follows:

$$\text{LHSV} = \frac{\text{Volume Of Total Liquid Feed Run Through The Reactor Per Hour}}{\text{Volume of Catalyst in Reactor}}$$

The examples which follow illustrate the synthesis of ethylene and propylene glycol ethers from low molecular weight olefins and the corresponding glycol using heteropoly acid catalysts.

Conversion of glycol (wt%) is estimated in the following examples using the equation:

$$\frac{\text{Wt\% Conc. of [Glycol in Feed - Glycol in product]}}{\text{Wt\% Conc of Glycol in Feed}} \times 100$$

Selectivities to glycol monoalkyl ether (mole%) are estimated from:

$$\frac{\text{Moles of Glycol Monoalkyl Ether Produced}}{\text{Moles of Glycol Converted}} \times 100$$

The accompanying examples illustrate:

(1) The synthesis of ethylene glycol t-butyl ether from ethylene glycol plus isobutylene using heteropoly acid catalysts (e.g. 12-tungstophosphoric acid) at 120 °C (Example 1).

(2) Similar preparations of ethylene glycol isopropyl ether at a higher temperature starting from ethylene glycol plus propylene (Example 2).

(3) The preparation of propylene glycol mono-t-butyl ethers from propylene glycol plus isobutylene, again using the 12-tungstophosphoric acid catalysts (Example 3).

(4) The preparation of ethylene glycol mono-t-butyl ether from ethylene glycol plus isobutylene using a series of heteropoly acid catalysts, including 12-molybdophosphoric acid, 12-tungstosilicic acid and 12-molybdosilicic acid (see Examples 4-6).

(5) The generation of propylene glycol mono-t-butyl ether in continuous unit equipment starting from a homogeneous mix of 1,2-propylene glycol and isobutylene over a range of operating temperatures and space velocities with 12-tungstophosphoric acid and 12-molybdophosphoric acid as the solubilized catalysts. At 100 °C, propylene glycol monoalkyl ether selectivity is estimated to be 92 mole% (see Example 7) and >99 mole% (Example 11) respectively.

(6) The formation of the two propylene glycol mono-isopropyl ethers, 1-isopropoxy-2-propanol and 2-isopropoxy-1-propanol, from propylene glycol plus propylene again in the presence of soluble 12-tungstophosphoric acid as catalyst (Examples 8-10).

(7) The generation of propylene glycol mono-t-butyl ether in continuous unit equipment using a heterogeneous 12-molybdophosphoric acid-on-titania catalyst (Example 12).

## EXAMPLE 1

### Synthesis of Ethylene Glycol Mono-t-Butyl Ether

To a 300 cc pressure reactor fitted with heating, mixing and temperature controls was charged a mixture of ethylene glycol (62.0g, 1.0 mole) and 12-tungstophosphoric acid (5.0g). The reactor was flushed with nitrogen and pressured with isobutylene (28.0g, 0.5 mole), then heated to 120 °C with mixing. After 4 hours at temperature, the reactor was cooled and the product (92.5g) recovered as a water-white liquid. Analysis by GC and GC-IR showed the presence of:

| | |
|---|---|
| Ethylene glycol mono-t-butyl ether | 54.3% |
| Ethylene glycol di-t-butyl ether | 11.6% |
| Ethylene glycol | 25.4% |
| Isobutylene | 6.7% |
| t-butanol | 1.2% |

Ethylene glycol conversion is 64%. Ethylene glycol mono-t-butyl ether selectivity is 73%.

## EXAMPLE 2

### Synthesis of Ethylene Glcyol Isopropyl Ether

To a 300 cc pressure reactor fitted with heating, mixing and temperature controls was charged a mixture of ethylene glycol (62.0g, 1.0 mole) and 12-tungstophosphoric acid (1.0g). The reactor was flushed with nitrogen and pressured with propylene (21.0g, 0.5 mole), then heated to 180 °C with mixing. After 4 hours at temperature, the reactor was cooled and the product (70.0g) recovered.

Analysis of the clear liquid product by GC and GC-IR showed the presence of:

| | |
|---|---|
| Ethylene glycol isopropyl ether | 32.7% |
| Ethylene glycol di-isopropyl ether | 2.1% |
| Ethylene glycol | 54.0% |
| Diethylene glycol | 6.5% |

## EXAMPLE 3

### Synthesis of Propylene Glycol Mono-t-Butyl Ether

To a 300 cc pressure reactor fitted with heating, mixing and temperature controls, was charged a mixture of propylene glycol (76.1g, 1.0 mole) and 12-tungstophosphoric acid (1.0g). The reactor was flushed with nitrogen and pressured with isobutylene (28.0g, 0.5 mole), then heated to 120° with mixing. After 4 hours at temperature, the reactor was cooled and the product (105.3g) recovered as a water-White liquid.

Analysis by GC and GC-IR showed the presence of:

| Propylene glycol mono-t-butyl ethers | |
|---|---|
| 1-(t-butoxy)-2-propanol | 30.9% |
| 2-(t-butoxy)-2-propanol) | 3.8% |
| Propylene glycol di-t-butyl ether | 5.3% |
| Propylene glycol | 38.2% |
| Isobutylene | 4.1% |

## EXAMPLES 4-6

### Synthesis of Ethylene Glycol Mono-t-Butyl Ethers

Following the procedures of Example 1, ethylene glycol (62.0g, 1.0 mole) and isobutylene (28.0g, 0.5 mole) were reacted in the presence of a series of heteropoly acids (5.0g) at 120 °C for 4 hours.

Analyses of the product solutions by GC-IR showed the presence of:

| Example | Catalyst | t-Butoxy Ethanol | Dibutoxy Ethane | Isobutylene | EG | t-Butanol |
|---|---|---|---|---|---|---|
| 4 | $H_3PO_4 \cdot 12MoO_3$ | 47.8 | 12.0 | 10.5 | 24.3 | 5.1 |
| 5 | $H_4SiO_4 \cdot 12WO_3$ | 26.9 | 7.5 | 22.9 | 27.3 | 1.8 |
| 6 | $H_4SiO_4 \cdot 12MoO_3$ | 44.0 | 12.8 | 15.7 | 21.3 | 3.8 |

It is evident from the data that all three heteropoly acids 12-molybdophosphoric acid (Example 4), 12-tungstosilicic acid (Example 5) and 12-molybdosilicic acid (Example 6) are effective catalysts for the subject synthesis.

## EXAMPLE 7

### Synthesis of Propylene Glycol Mono-t-Butyl Ethers

To a 50 cc capacity, plug flow, continuous reactor fitted with temperature and pressure controls, was charged a single-phase liquid mixture comprising:
Isobutylene (2267g, 40.5 mole)
1,2-propylene glycol (2933g, 38.5 mole)
p-Dioxane (4000g)
12-tungstophosphoric acid (40.0g)

This mixture was fed to the reactor at a rate of 40 cc/hr, with a back pressure of 300 psi, and heated to a set temperature. After allowing the system to equilibrate overnight, two effluent samples were collected in 316 ss pressure bombs.

The procedure was repeated at four different operating temperatures (60-120.C) and three feed rates (40-200 cc/hr, LHSV = 1-5).

Analyses of these on-line product mixtures are summarized in Table 2. For sample #1, operated at 100.C.
Propylene glycol conversion is 56%.
Propylene glycol mono-t-butyl ether selectivity (basis PG converted) is 92 mole%.
Ratio of mono-t-butyl ethers, 2-OH/1-OH = 8.3

## EXAMPLE 8

### Synthesis Of Propylene Glycol Mono-iso-Propyl Ethers

To a 300 cc pressure reactor of Example 1 was charged a mixture of 1,2-propylene glycol (76.1g, 1.0 mole) and 12-tungstophosphoric acid (5.0g). The reactor was flushed with nitrogen and pressured with propylene (21.0g, 0.5 mole), then heated to 180 °C with mixing after 4 hours at temperature, the reactor was cooled and the product (96.0g) recovered as a deep red liquid.

Analysis by GC and GC-IR showed the presence of:

| | |
|---|---|
| 1-isopropoxy-2-propanol | 13.7% |
| 2-isopropoxy-1-propanol | 2.9% |

TABLE 2

1,2-PROPYLENE GLYCOL + ISOBUTYLENE (1:1.05)

| Catalyst | (°C) | Temp. cc/hr | Flow Day | Sample | $C_4^=$ | p-Dioxane | PG | PG Di-Bu Ether | PG-BuEther 1-OH | PG-BuEther 2-OH |
|---|---|---|---|---|---|---|---|---|---|---|
| | | | | | ← Conc. (Wt%) → | | | | | |
| W-P[a] | | | | FS | 24.5 | 43.2 | 30.1 | 0.1 | 0.8 | 1.2 |
| | 100 | 40 | 1 | 1 | 7.6 | 40.0 | 13.3 | 7.2 | 2.9 | 24.0 |
| | | | | 2 | 7.5 | 40.2 | 13.9 | 7.1 | 2.8 | 23.8 |
| | 60 | 40 | 2 | 3 | 16.0 | 41.6 | 22.8 | 2.3 | 4.1 | 8.7 |
| | | | | 4 | 16.6 | 41.9 | 24.2 | 1.8 | 4.1 | 8.4 |
| | 80 | 40 | 3 | 5 | 8.1 | 40.5 | 12.4 | 10.0 | 3.8 | 23.1 |
| | | | | 6 | 8.2 | 40.5 | 13.3 | 9.4 | 3.9 | 22.7 |
| | 120 | 40 | 4 | 7 | 7.4 | 39.2 | 16.8 | 4.3 | 2.5 | 18.4 |
| | | | | 8 | 8.2 | 39.4 | 17.6 | 3.5 | 2.3 | 18.3 |
| | 100 | 100 | 5 | 9 | 6.0 | 39.4 | 11.6 | 9.3 | 3.4 | 23.7 |
| | | | | 10 | 7.1 | 40.1 | 13.8 | 7.3 | 3.1 | 23.0 |
| | 100 | 200 | 6 | 11 | 7.8 | 39.2 | 15.1 | 5.9 | 2.7 | 21.1 |
| | | | | 12 | 7.3 | 39.4 | 15.3 | 5.7 | 2.7 | 20.9 |

[a] 12-Tungstophosphoric acid solubilized in feed mix (FS)

## EXAMPLES 9 AND 10

### Synthesis of Propylene Glycol Mono-Isopropyl Ethers

Following the procedures of Example 8, the mix of 1,2-propylene glycol (76.1g, 1.0 mmole), 12-tungstophosphoric acid (5.0g) and propylene (21.0g, 0.5 mole) were heated to 150°C and 120°C for 4 hours in separate experiments.

The total liquids recovered and the concentrations of propylene glycol mono-iso-propyl ethers in those product samples are tabulated below:

| Example | Reaction Temp(°C) | Total Liquid Product(g) | Concentration (%) Of: 1-Isopropoxy 2-Propanol | Concentration (%) Of: 2-Isopropoxy 1-Propanol |
|---|---|---|---|---|
| 9 | 150 | 90.4 | 15.8 | 9.5 |
| 10 | 120 | 80.5 | 3.5 | 2.9 |

## EXAMPLE 11

### Synthesis Of Propylene Glycol Mono-t-Butyl Ethers

Following the continuous preparative procedures of Example 7, the 50 cc capacity continuous reactor was charged with a single-phase liquid mixture comprising:
Isobutylene (2267g, 40.5 mole)
1,2-propylene glycol (2933g, 38.5 mole)
p-Dioxane (4000g)
12-molybdophosphoric acid (40.0g)

The mix was fed over a range of feed rates (40-200 cc/hr, 1HSV = 1-5) using four different operating temperatures (60-120 °C). Analyses of the on-line product mixtures are summarized in Table 3. For Sample #1, operated at 100 °C:
Propylene glycol conversion is 46%.
Propylene glycol mono-t-butyl ether selectivity (basis PG converted) is >99 mole%.
Ratio of mono-t-butyl ethers, 2-OH/1-OH = 7.7.

TABLE 3

1,2-PROPYLENE GLYCOL + ISOBUTYLENE (1:1.05)

| Catalyst | Temp. (°C) | Flow (cc/hr) | Day | Sample | Conc. (Wt) $C_4^=$ | p-Dioxane | PG | PG Di-Bu | PGBuEther 1-OH | PGBuEther 2-OH |
|---|---|---|---|---|---|---|---|---|---|---|
| Mo-P[a] | | | | FS | 20.2 | 41.9 | 26.9 | 1.1 | 1.1 | 1.8 |
| | 100 | 40 | 1 | 1 | 10.2 | 42.2 | 15.9 | 5.2 | 2.7 | 20.7 |
| | | | | 2 | 11.9 | 42.2 | 17.6 | 4.4 | 2.7 | 19.1 |
| | 60 | 40 | 2 | 3 | 18.7 | 43.2 | 26.9 | 1.5 | 1.9 | 6.6 |
| | | | | 4 | 21.6 | 43.9 | 28.4 | 0.4 | 1.3 | 2.8 |
| | 80 | 40 | 3 | 5 | 19.6 | 43.5 | 26.6 | 1.0 | 2.4 | 4.9 |
| | | | | 6 | 17.7 | 42.3 | 25.1 | 1.3 | 2.4 | 4.9 |
| | 120 | 40 | 4 | 7 | 14.5 | 41.9 | 18.9 | 3.0 | 2.5 | 17.2 |
| | | | | 8 | 14.9 | 41.8 | 19.1 | 2.9 | 2.5 | 16.7 |
| | | | | FS-2 | 25.7 | 42.5 | 29.4 | 0.1 | 0.7 | 1.0 |
| | 100 | 100 | 5 | 9 | 19.4 | 41.9 | 23.7 | 1.4 | 2.5 | 9.6 |
| | | | | 10 | 18.7 | 41.8 | 24.3 | 1.4 | 2.5 | 8.1 |
| | 100 | 200 | 6 | 11 | 23.3 | 42.5 | 28.0 | 0.2 | 1.4 | 2.8 |
| | | | | 12 | 23.1 | 41.8 | 26.9 | 0.2 | 1.4 | 2.8 |

[a] 12-Molybdophosphoric acid solubilized in feed mix (FS)

## EXAMPLE 12

**Synthesis Of Propylene Glycol Mono-t-Butyl Ethers**

To a 50 cc capacity, plug-flow, continuous reactor fitted with temperature and pressure controls was charged 40 cc of a 12-molybdophosphoric acid-on-titania catalyst (3.1% molybdenum, 1/8" titania extrudates from the Norton Company). The reactor was fed a single-phase liquid mixture of:

Isobutylene (2267g, 40.5 mole)
1,2-propylene glycol (2933g, 38.5 mole)
p-Dioxane (4000g)

at a rate of 40 cc/hr, with a back pressure of 300 psi, and an operating temperature of 100°C. After allowing the system to equilibrate, two effluent samples were collected in 316 ss pressure bombs.

Analyses of these or-line product mixtures were typically:

| | |
|---|---|
| Isobutylene | 15.2% |
| p-Dioxane | 41.5% |
| 1,2-Propylene Glycol | 18.4% |
| 1-t-Butoxy-2-Propanol | 16.1% |
| 2-t-Butoxy-1-Propanol | 2.2% |
| 1,2-Dibutoxy Propane | 3.3% |

## Claims

1. A method for the synthesis of ethers of polyols by reacting an olefin and a polyol in the presence of a catalyst characterized in that the catalyst is a heteropoly acid and reaction is carried out at a temperature of 25 to 200°C and a pressure of up to 7MPa.

2. A method according to Claim 1 characterized in that the heteropoly acid has the Keggin structure, $H_{8-n}[XM_{12}O_{40}]$, where X is P or Si, M is Mo or W, and n is 4 or 5.

3. A method according to 1 or 2 characterized in that the heteropoly acid is 12-tungstophosphoric acid, 12-molybdophosphoric acid, tungstosilicic acid or 12-molybdosilicic acid.

4. A method according to any one of Claims 1 to 3 characterized in that reaction is conducted in the pressure of an inert solvent.

5. A method according to any one of Claims 1 to 3 characterized in that the heteropoly acid catalyst is solubilized in the olefin and polyol reactants.

6. A method according to any one of Claims 1 to 3 characterized in that the heteropoly acid is supported on an inert support.

7. A method according to Claim 6 characterized in that the inert support is titanium dioxide, alumina or silica.

8. A method according to Claim 6 or 7 characterized in that the concentration of the polyatom, M, in the formulated catalyst is from 0.1 to 30 wt%.

9. A method according to any one of Claims 1 to 8 characterized in that the olefin has the formula:

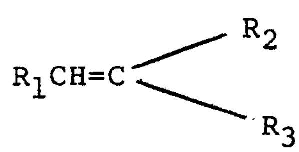

wherein $R_1$, $R_2$, and $R_3$ are each hydrogen or alkyl, and $R_1 + R_2 + R_3$ contain a total of 0 to 4 carbon atoms.

10. A method according to Claim 9 characterized in that the olefin is propylene or isobutylene.

11. A method according to any one of Claims 1 to 10 characterized in that the polyol is a glycol having 2 to 20 carbon atoms.

12. A method according to Claim 11 characterized in that the glycol is ethylene glycol, 1,2-propylene glycol, 1,2-butylene glycol, diethylene glycol or triethylene glycol.